# EUROPEAN PATENT SPECIFICATION

(11) **EP 1 803 442 B1**
(45) Date of publication and mention of the grant of the patent: **10.02.2010**
(21) Application number: 06255568.5
(22) Date of filing: 30.10.2006
(51) Int. Cl.: A61K 9/16

(54) **Mineralized hydrogels and methods of making and using mineralized hydrogels**
Mineralisierte Hydrogele und Verfahren zur Herstellung und Verwendung mineralisierter Hydrogele
Hydrogels mineralisés et procedures pour la production et l'utilisation des hydrogels mineralisés

(30) Priority: 28.10.2005 US 731092 P; 27.10.2006 US 553830
(43) Date of publication of application: 04.07.2007
(73) Proprietor: ZIMMER INC., Warsaw, Indiana 46580-2746 (US)
(72) Inventor: Zhang, Kai, Warsaw, Indiana 46582 (US); Hawkins, Michael E., Colombia City, Indiana 46725 (US); Thomas, Brian, Colombia City, Indiana 46725 (US); Brinkerhuff, Hallie, Winona Lake, Indiana 46590 (US)
(74) Representative: Findlay, Alice Rosemary

(56) References cited:
- EP-A1- 1 595 899
- WO-A-2007/015208
- FR-A- 2 865 939
- US-A- 5 122 565

## Description

The present invention relates to mineralized hydrogels suitable for use in biomedical or other applications

Hydrogels are water-swellable or water-swollen materials having a structure defined by a crosslinked network of hydrophilic homopolymers or copolymers. The hydrophilic homopolymers or copolymers may be water-soluble in free form, but in a hydrogel are rendered insoluble (but water-swellable) in water due to covalent, ionic, or physical crosslinking. In the case of physical crosslinking, the linkages may take the form of entanglements, crystallites, or hydrogen-bonded structures. The crosslinks in a hydrogel provide structure and physical integrity to the network.

Hydrogels may be classified as amorphous, semicrystalline, hydrogen-bonded structures, supermolecular structures, or hydrocolloidal aggregates. Numerous parameters affect the physical properties of a hydrogel, including porosity, pore size, the hydrogel used, molecular weight of gel polymer, and crosslinking density. The crosslinking density, for example, influences the hydrogel's macroscopic properties, including volumetric equilibrium swelling ratio (denoted "Q"), compressive modulus, and mesh size (ζ), which is the space between macromolecular chains in a cross-linked network usually characterized by the distance between adjacent cross-links. Hydrogels in Medicine and Pharmacy, CRC Press, 1986, edited by Nicholas A. Peppas. Pore size and shape, pore density, and other factors can impact the surface properties, optical properties, and mechanical properties of a hydrogel.

Hydrogels have been derived from a variety of hydrophilic polymers and copolymers. Poly(vinyl alcohol) ("PVA"), Poly(ethylene glycol), poly(vinyl pyrrolidone), polyacrylamide, poly(hydroxyethyl methacrylate) ("PHEMA"), and copolymers of the foregoing, are examples of polymers from which hydrogels have been made. Hydrogels have also been formed from biopolymers such as chitosan, agarose, hyaluronic acid and gelatin, as well as (semi) interpenetrating network ("IPN") hydrogels such as gelatin crosslinked with poly(ethylene glycol) diacrylate.

Hydrogels have shown promise in biomedical and pharmaceutical applications, mainly due to their high water content and rubbery or pliable nature, which can mimic natural tissue and can facilitate the release of bioactive substances at a desired physiological site. For example, hydrogels have been used and/or proposed in a variety of tissue treatment applications, including implants, tissue adhesives, and bone grafts for spinal and orthopedic treatments such as meniscus and articular cartilage replacement. One drawback to the use of conventional hydrogels in certain tissue treatment applications, and in particular bone tissue treatments, is that such hydrogels do not necessarily provide an optimal scaffolding for encouraging tissue growth and/or formation of calcified tissues. For example, conventional hydrogels do not have substantial osteoconductive characteristics, and therefore, do not suitably encourage the formation of bone tissue, either on the surface or within such hydrogel materials. Conventional hydrogels may also lack suitable mechanical properties, e.g. strength, for certain tissue treatments, in particular calcified and/or bony tissue treatments.

In an attempt to improve the osteoconductive characteristics and/or mechanical properties of hydrogels, calcium phosphate minerals such as hydroxyapatite have been incorporated into previously-prepared hydrogels (e.g., PHEMA or PVA), for example, by soaking the hydrogel in a concentrated calcium phosphate solution such as a simulated body fluid, by alternately immersing the hydrogel in a calcium solution and a phosphate solution, and by physically mixing previously prepared hydrogels and calcium phosphate minerals.

Unfortunately, each of these approaches has certain drawbacks. The first two approaches, which involve immersing pre-formed hydrogels, may not provide suitable calcium phosphate mineral distribution within the hydrogel, and the mineralization process may be difficult to control. Additionally, in-situ (e.g., in a mold) reactions may not be achievable. With the third approach, the hydrogel may not suitably bind to the mineral, and it may be difficult to prepare articles with mineral concentration gradients. Consequently, these approaches to providing combining hydrogels with calcium phosphate minerals may have significant commercial limitations.

Therefore, it would be beneficial to provide methods of making and using mineralized hydrogels that overcome one or more of the aforementioned drawbacks.

The present invention provides methods of making and using mineralized hydrogels, in which calcium phosphate minerals are dispersed within a hydrogel polymer. In one embodiment, a calcium phosphate dispersion may first be formed by combining a first mixture including a calcium derivative, a second mixture including a phosphate derivative, and a hydrogel precursor to form a calcium phosphate dispersion containing the hydrogel precursor. The hydrogel precursor present in the calcium phosphate dispersion is then crosslinked to form a mineralized hydrogel, in which the calcium phosphate minerals may be substantially uniformly disposed within the mineralized hydrogel. Optional treatments may be employed to modify the calcium phosphate dispersion and/or minerals into a desired form such as a calcium-deficient apatite, which mimic biological bone and/or other calcified tissues.

The invention will now be described by way of example with reference to the accompanying drawings in which:
FIG. 1 is a flow-chart illustrating a method of forming mineralized hydrogels according to an embodiment of the present invention.
FIGS. 2A-2B illustrate an instrument for delivering mineralized hydrogels according to an embodiment of the present invention.
FIGS. 3A-3B are Fourier Transform Infrared Spectroscopy (FTIR) spectra of samples formed according to Example 1.
FIGS. 4A-4B are Scanning Electron Microscope (SEM) images of samples formed according to Example 1.
FIGS. 5A-5C are images of samples formed according to Example 4.
FIGS. 6A-6B are images of samples formed according to Example 5.

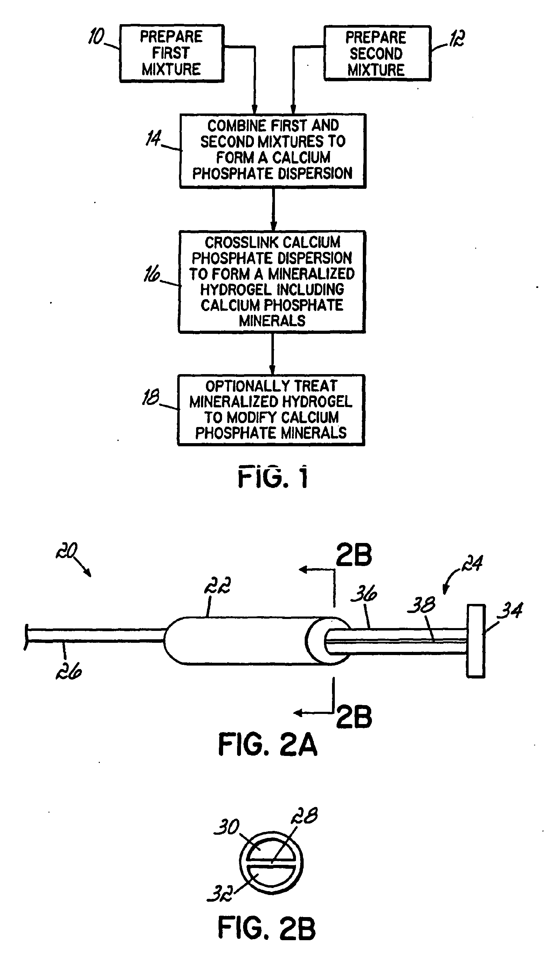

FIG. 1 is a flow-chart summarizing a method of forming a mineralized hydrogel according to one embodiment of the present invention. In 10 and 12, a first mixture including a calcium derivative and a second mixture including a phosphate derivative are separately prepared and combined in 14 to form a calcium phosphate dispersion. A hydrogel precursor may be added to either or both of the first and second mixtures prior to being combined. Alternatively, the hydrogel precursor may be added during or after the first and second mixtures are combined to form the calcium phosphate dispersion. In either case, in 16, the hydrogel precursor contained in the calcium phosphate dispersion is subsequently crosslinked to form a mineralized hydrogel including calcium phosphate minerals. In 18, optional pH treatments may also be carried out to modify the calcium phosphate mineral. Each of these steps is described in detail below.

### Preparation of First and Second Mixtures

In one embodiment, in 10, the first mixture includes a calcium derivative, an optional hydrogel precursor and an aqueous carrier (e.g., deionized water or miscible solutions of water and organic solvents). Suitable calcium derivatives include CaCl₂, Ca(OH)₂ and Ca(NO₃)₂. The calcium derivative may be combined with the aqueous carrier to form an aqueous calcium dispersion. The optional hydrogel precursor may be combined with the aqueous dispersion via conventional mixing methods.

In another embodiment, in 12, the second mixture includes a phosphate derivative, an optional hydrogel precursor and an aqueous carrier. Examples of suitable phosphate derivatives include K₂HPO₄, Na₂HPO₄, H₃PO₄ and (NH₄)₂HPO₄. The phosphate derivative may be combined with the aqueous carrier to form an aqueous phosphate dispersion. The optional hydrogel precursor may then be combined with the aqueous phosphate dispersion using conventional methods.

As used herein, the term "hydrogel precursor" refers to hydrogels and hydrogel polymer source materials (e.g. macromers, monomers, oligomers, homopolymers, copolymers, and/or (semi)-interpenetrating polymer networks (IPNs), which form hydrogels) that may be processed into a hydrogel. Suitable hydrogel precursors may be derived from a variety of polymer materials including poly(vinyl alcohol) ("PVA"), poly(glycols) such as poly(ethylene glycol) dimethacrylate ("PEGDMA"), poly(ethylene glycol) diacrylate ("PEGDA"), poly(hydroxyethyl methacrylate) ("PHEMA"), poly(vinyl pyrrolidone), poly(acrylamide), poly(acrylic acid), hydrolyzed poly(acrylonitrile), poly(ethyleneimine), ethoxylated poly(ethyleneimine) and poly(allylamine), polypeptides, as well as monomers, oligomers, macromers, copolymers and/or other derivatives of the foregoing. Biopolymers may also be used in certain embodiments. Suitable biopolymers include anionic biopolymers such as hyaluronic acid, cationic biopolymers such as chitosan, amphipathic polymers such as collagen, gelatin and fibrin, and neutral biopolymers such as dextran and agarose. Interpenetrating polymer networks (e.g. combinations of water soluble polymers and water insoluble polymers), polymers modified or grafted with (poly)peptides or proteins, and polymers having backbones modified with calcium or phosphate derivatives may also be suitable for use in certain embodiments. Additional polymers which may be suitable for use in certain embodiments are reported in U.S. Patent No. 6,224,893 to Langer et al. Suitable hydrogel blends are reported in U.S. Application No. 11/358,383 entitled "Blend Hydrogels and Methods of Making.

If a hydrogel precursor is added to both the first and second mixtures, the hydrogel precursor used in each mixture may be the same hydrogel precursor. Alternatively, the hydrogel precursors may be different than, but capable of crosslinking with, one another. In one embodiment, about 10 w/w% hydrogel precursor may be added to each mixture.

In certain embodiments of the present invention, the hydrogel precursor is poly(vinyl alcohol), or a derivative thereof. Poly(vinyl alcohol) may be produced by free-radical polymerization of vinyl acetate to form poly(vinyl acetate), followed by hydrolysis to yield PVA. The hydrolysis reaction does not go to completion, which leaves pendent acetate groups at some points along the polymer chain. The extent of the hydrolysis reaction determines the degree of hydrolysis of the PVA. Commercially available PVA can have a degree of hydrolysis over 98% in some cases. PEGDA and PEGDMA are also suitable for use in particular embodiments.

In certain embodiments, ions such as F⁻, Cl⁻, Na⁺, K⁺, Mg²⁺, Sr²⁺, Ba²⁺, CO₃²⁻, and/or SO₄²⁻ (or salts thereof) may be added to the first and/or second mixture to form calcium deficient apatites when the first and second mixtures are combined as reported in greater detail below.

Optionally, a radiation sensitive material such as a photoinitiator may be added to the first and/or second mixture (or subsequently to the calcium phosphate dispersion) to facilitate crosslinking of the hydrogel precursor as reported below. IRGACURE^{®} brand photoinitiators (available from Ciba Specialty Chemicals) is an example of a group of suitable radiation sensitive materials. Other optional additives include biocompatible preservatives, surfactants, colorants and/or other additives conventionally added to polymer mixtures.

The properties and/or physical characteristics of the first and second mixtures may vary depending on the type and amount of carrier and the type and concentration of hydrogel precursor added to the carrier. In certain embodiments, the mixtures will have characteristics similar to a traditional solution In other embodiments, for example embodiments utilizing PVA hydrogel precursors, the mixture may take on characteristics (e.g. water swellable) similar to a hydrogel without any additional crosslinking steps.

### Combining First and Second Mixtures

In 14, the first and second mixtures may be combined under conditions suitable to form a calcium phosphate dispersion including calcium phosphate minerals (generally nano- to micro- sized particles formed from precipitation) into which the hydrogel precursor is dispersed, dissolved or are otherwise contained. As used herein, the phrase "calcium phosphate dispersion" broadly encompasses dispersions, slurries, solutions, and any other mixture formed by combining the calcium and phosphate derivatives reported herein. The conditions under which the first and second mixtures are combined may affect the resulting reaction between the calcium and phosphate derivatives.

The order in which the first and second mixtures are combined may be varied. In one embodiment, the second mixture is added to the first mixture at a controlled rate and under continuous agitation to form the desired calcium phosphate dispersion. In another embodiment, the first mixture is added to the second mixture at a controlled rate and under continuous agitation to form the desired calcium phosphate dispersion.

Reaction temperature may also be varied. In one embodiment, the mixtures may be combined at room temperature, i.e., at a temperature of about 25 °C. In another embodiment, the mixtures may be combined at physiological temperature, i.e., at a temperature of about 37 °C. In another embodiment, the mixtures may be combined at a temperature between about 25 °C and about 37 °C.

Additional conditions that may be varied include the atmosphere (e.g., air, inert or CO2), the concentration of the calcium and or phosphate derivative in each mixture, the calcium:phosphate ion ratio, and the pH of the first and second mixtures. These conditions may affect the calcium phosphate mineral or minerals formed when the first and second mixtures are combined. The types of calcium phosphate minerals that may be formed include amorphous calcium phosphates, dicalcium phosphate dihydrate, tricalcium phosphate, octacalcium phosphate, calcium deficient apatites and hydroxyapatite.

Amorphous calcium phosphate minerals formed according to embodiments of the present invention do not show traditional crystalline peaks from X-ray diffraction (XRD) characterization and may not have a fixed chemical structure, but instead are defined by their substantially non-crystalline and/or nano-crystalline structure.

Dicalcium phosphate dehydrate formed according to embodiments of the present invention has the following formula:

CaHPO₄ • 2H₂O

Calcium-deficient apatites formed according to embodiments of the present invention have the following formula:

(Ca, M)₁₀(PO₄, CO₃, X)₆(OH, F, Cl)₂

wherein M includes minor ions and/or trace elements such as Na⁺, K⁺, Mg²⁺,Sr²⁺, Ba²⁺, and X is BPO₄²⁻ or SO₄² The presence of minor ions in calcium deficient apatites may be achieved by adding the ion (such as in the form of a salt) to the first and/or second mixture.

Hydroxyapatite formed according to embodiments of the present invention has the following formula:

Ca₁₀(PO₄)₆(OH)₂

In one embodiment, the concentration of the calcium ions in the first mixture and the phosphate ions in the second mixture may be between about 0.1 mM and about 5M, more particularly between about 0.5M and about 2M. In another embodiment, the first mixture includes about a 1 M concentration of calcium ions and the second mixture includes about a 0.67M concentration of phosphate groups.

The resulting ratio of Ca ions to phosphate ions in the first and second mixtures may range from about 1:1 to about 2:1. Ratios between about 1:1 and about 1.5:1 may encourage the formation of amorphous calcium phosphate minerals. Ratios between about 1.5:1 and 2:1, more particularly between about 1.5:1 and about 1.67:1, may encourage the formation of calcium-deficient minerals.

When the first and second mixtures are combined in a manner that encourages the formation of amorphous calcium phosphate, but a more crystalline form of calcium phosphate (e.g. calcium-deficient apatite) is desired, the calcium phosphate dispersion may be further treated in a pH environment suitable for transforming the amorphous calcium phosphate to a crystalline form. In one embodiment, the pH of the calcium phosphate dispersion may be adjusted to between about 6.5 and 7.5 (i.e. approximately neutral pH), more particularly between about 7.2 and about 7.4 to encourage transformation from amorphous calcium phosphate to a crystalline form. In another embodiment, the pH may be adjusted to a pH of greater than 7.5 (i.e. a basic dispersion), more particularly between about 10 and 11 to transform amorphous calcium phosphate to a crystalline form at a different reaction rate and/or kinetics. For example, the calcium phosphate dispersion may be combined with a basic solution such as an ammonia solution or a sodium hydroxide solution, or a buffer solution such as a phosphate buffered saline, or a tris-buffer. Additional description of the conversion of amorphous calcium phosphate to crystalline calcium phosphate may be found in LeGeros R.Z., "Calcium phosphates in oral biology and medicine," Monograph in Oral Science, Vol. 15, pp. 1-201, and Chow et al., "Octacalcium Phosphate," Monograph in Oral Science, Vol. 18, pp. 94-112 and 130-148. In alternative embodiments, similar pH treatments may also be employed after the formation of the mineralized hydrogel.

In one embodiment, the calcium phosphate dispersion is formed and/or treated to encourage the formation of calcium-deficient apatite. One benefit of calcium-deficient apatite as opposed to other calcium phosphate minerals is that calcium deficient apatite is known to more closely mimic biological apatite in the body, and may therefore provide improved osteoconductive properties compared to other calcium phosphate minerals.

The properties and/or physical characteristics of the mixture will also affect the characteristics of the calcium phosphate dispersion. In certain embodiments the calcium phosphate dispersion will have characteristics similar to a traditional dispersion. In other embodiments, for example embodiments utilizing PVA hydrogel precursors, the calcium phosphate dispersion may take on characteristics (e.g. water swellable) similar to a hydrogel without additional crosslinking steps.

Prior to crosslinking the hydrogel precursor as reported below, the calcium phosphate may be subjected to continuous mixing to provide a substantially uniform dispersion. Alternatively, the dispersion could be placed in a centrifuge device, and subjected to a sufficient force to create a calcium phosphate mineral gradient in the dispersion.

### Crosslink Hydrogel Precursor

After forming the desired calcium phosphate dispersion, in 16, the hydrogel precursor in the dispersion is optionally crosslinked to form a mineralized hydrogel, in which the calcium phosphate minerals are dispersed and/or distributed within the mineralized hydrogel. A variety of approaches may be used to crosslink the hydrogel precursor, including photoinitiation, irradiation, physical crosslinking (e.g., freeze thaw method), and chemical crosslinking.

In one embodiment of the present invention, crosslinking is achieved by exposing the calcium phosphate dispersion to ultraviolet or visible blue light (collectively referred to herein as "UV/Vis radiation"). In this embodiment, a photoinitiator such as an IRGACURE brand initiator may be combined with the calcium phosphate dispersion, or with one of the mixtures used to form the calcium phosphate dispersion. Suitable UV/Vis radiation sources according to one embodiment may operate at a wavelength ranging between about 200 to 700 nm, more particularly between about 320 nm and about 700 nm, and even more particularly between about 350 and about 520 nm. Suitable energy levels for the UV/Vis radiation source may range between about 10 µW/cm² and about 20 W/cm². In a particular embodiment, a wavelength of approximately 365 nm and an energy level of 300 µW/cm² may be suitable. Crosslinking by electron-beam or gamma irradiation, such as by using a Co⁶⁰ source, may also be employed according to embodiments of the present invention.

In another embodiment, physical crosslinking may be achieved by conventional freeze-thaw techniques, for example, as described in Peppas, et al., Adv. Polymer Sci. 153, 37 (2000).

Examples of suitable chemical crosslinking agents for addition to the calcium phosphate dispersion (or the first or second mixture) include monoaldehydes such as formaldehyde, acetaldehyde, or glutaraldehyde in the presence of a solvent such as methanol. Other suitable crosslinking agents include diisocyanate compounds, which can result in urethane linkages, or epoxy compounds. Crosslinking achieved using enzymes such as a calcium independent microbial transglutaminase, which catalyzes transamidation reactions to form N-ε-(γ-glutamyl)lysine crosslinks in proteins, may also be suitable according to embodiments of the present invention.

A combination of crosslinking techniques may also be utilized in the invention. For example, a freeze-thaw cycle could be used to provide physical crosslinking, followed by irradiation or photoinitiation to provide more complete crosslinking. As other examples, chemical crosslinking could be followed by irradiation or photoinitiation, or a freeze-thaw step could be performed after crosslinking by any of chemical, irradiation or photoinitiation.

The type, concentration and chemical properties (e.g. molecular weight) of the hydrogel precursor added to the first and/or second mixtures, as well as the employed crosslinking technique, may affect the properties and/or characteristics of the formed hydrogels. For example, swelling ratio, water content, mesh and/or pore size of the hydrogel may be varied by controlling the type, molecular weight and concentration of the hydrogel material. Also, the resulting hydrogel may be formed to be biodegradable or stable in vitro and/or in vivo by varying the chemistry of the hydrogel. For example, hydrogels formed from PEGDA are generally biostable, but hydrogels formed from copolymers of Poly(lactic acid) and PEGDA may be formed with controllable degradation properties (See, e.g., Anseth, et al., Journal of Controlled Release, 78 (2002), 199-209).

Additionally, the type of hydrogel precursor used may result in enhanced properties such as adhesion. For example, PEGDA, interpenetrating networks of PEGDA, and gelatins (including gelatin crosslinked by Ca-independent enzymes) may have enhanced or improved hydrogel adhesion properties. The addition of non-hydrogel phases or fillers (e.g. solid fillers) into the hydrogel, either prior to or after crosslinking, may also modify the properties of the hydrogel. The employed method of crosslinking the hydrogel precursor to form a mineralized hydrogel may also affect hydrogel properties. In certain embodiments, additional bioactive agents may be incorporated into the hydrogel prior to or after the hydrogel is formed. Examples of suitable bioactive agents include, without limitation, proteins such as bone morphogenic proteins ("BMPs"), growth factors, pharmaceuticals such as analgesics or antibiotics, enzymes and/or genes.

As previously noted, certain embodiments of the present invention may form a mineralized hydrogel material without additional crosslinking steps. For example, certain PVA polymers may form a hydrogel when the first and second mixtures are prepared. In such cases, a final crosslinking step is optional, but may further improve certain characteristics of the mineralized hydrogel.

In the foregoing manner, the form of the mineralized hydrogel may be varied depending on the specific application and the desired result. In certain applications for example, it may be desirable to disperse and/or distribute the calcium phosphate derivate in a substantially uniform and/or homogenous manner throughout the mineralized hydrogel. In these applications, a bulk mineralized hydrogel may be formed as reported herein by combining the first and second mixtures and crosslinking. Alternatively, the first and second mixtures may be combined in a desired mold to form an implant.

In other embodiments, a surface region having a uniform distribution of the calcium phosphate minerals, or a concentration gradient of the calcium phosphate minerals may be desirable. This could be accomplished by applying multiple layers of separately formed mineralized hydrogels having varying forms or concentrations of calcium phosphate minerals. Still further, it may be desirable to adhere layers of mineralized hydrogels with layers of conventional hydrogels.

The mineralized hydrogels of the present invention may be used in a wide range of medical applications, including orthopedic and spinal uses as implants and/or implant coatings, bone grafts and or bone cements, adhesive/fixation materials, orthobiologics (e.g. tissue engineering scaffolds and constructs), mineralized fillers, drug, growth factor and gene delivery, and in dental applications. In one embodiment, the mineralized hydrogels could be used to provide artificial articular cartilage as described, e.g., by Noguchi, et al., J. Appl. Biomat. 2, 101 (1991), or as artificial meniscus or articular bearing components. The hydrogels could also be employed to repair or replace the temporomandibular joint, proximal interphalangeal joint, metacarpophalangeal joint, metatarsalphalanx joint, or in hip capsule joint repair.

In another embodiment, the mineralized hydrogels of the present invention may be used to replace or rehabilitate the nucleus pulposus of an intervertebral disc. Degenerative disc disease in the lumbar spine is marked by a dehydration of the intervertebral disc and loss of biomechanical function of the spinal unit. A recent approach has been to replace only the central portion of the disc, called the nucleus pulposus. The approach entails a less invasive posterior surgery, and can be done rather rapidly. Bao and Higham developed a PVA hydrogel suitable for nucleus pulposus replacement, as reported in U.S. Pat. No. 5,047,055. The hydrogel material, containing about 70% water, acts similarly to the native nucleus, in that it absorbs and releases water depending on the applied load.

The mineralized hydrogels of the invention may also be employed in a spinal disc prosthesis used to replace a part of or all of a natural human spinal disc. By way of example, a spinal disc prosthesis may comprise a flexible nucleus, a flexible braided fiber annulus, and end-plates. The hydrogel may be employed in the flexible nucleus, for example. A spinal disc prosthesis is described in U.S. Pat. No. 6,733,533 to Lozier, for instance.

According to one embodiment, the mineralized hydrogels of the present invention may be formed into an implantable prosthetic device of the types reported herein. Such an implant may be formed by initially placing the first mixture in an implant mold. The second mixture may then be rapidly mixed and/or injected into the first mixture. The resulting calcium phosphate dispersion may then be crosslinked according to one of the methods reported herein to form a mineralized hydrogel implant. Alternate processing methods include solution casting, injection molding, or compression molding. In general, these methods may be used prior to or after crosslinking.

According to another embodiment of the present invention, a mineralized hydrogel may be delivered to a physiological site prior to, coincident with, or immediately after combining the first and second mixtures. FIGS. 2A-2B are schematic illustrations of an instrument 20 capable of combining the first and second mixtures and delivering the mixtures to a physiological site. The instrument 20 includes a syringe 22, a plunger 24, and a cannula 26. The syringe 22 includes a divider 28 for separating the first and second mixtures into respective reservoirs 30, 32. The plunger 24 includes a button 34, a shaft 36 having a channel 38, which slides relative to the divider 28 to deliver the first and second mixtures into the cannula 26. In this embodiment, the first and second mixtures first initially combine in the cannula 26.

In an alternate embodiment, separate plungers 24 for the first and second mixtures could be utilized such that the first and second mixtures could be delivered separately. In a further embodiment, the syringe 22 and/or cannula 26 could have a dual lumen construction such that the first and second mixtures do not contact until after exiting the cannula 26. The water-swellable material can then be crosslinked and/or hydrated after delivery and formation to provide a hydrogel.

The following examples are provided to illustrate the invention and are not intended to limit the same.

### EXAMPLE 1A

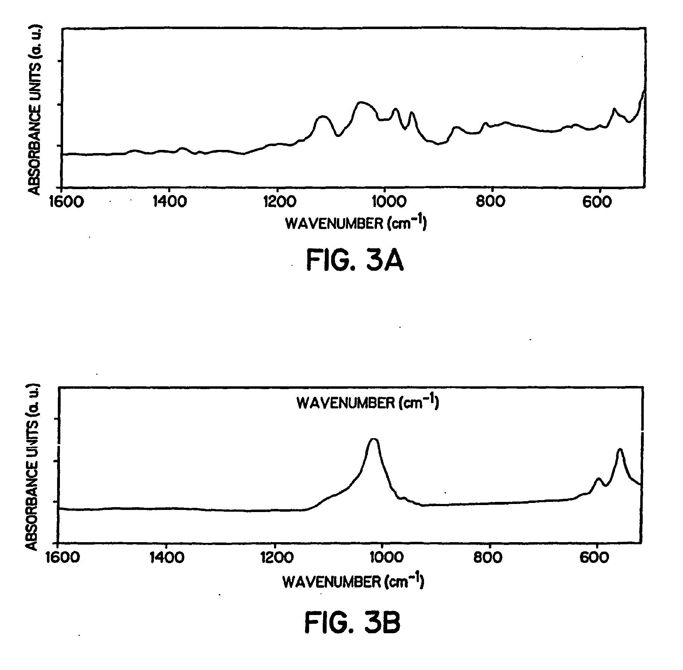

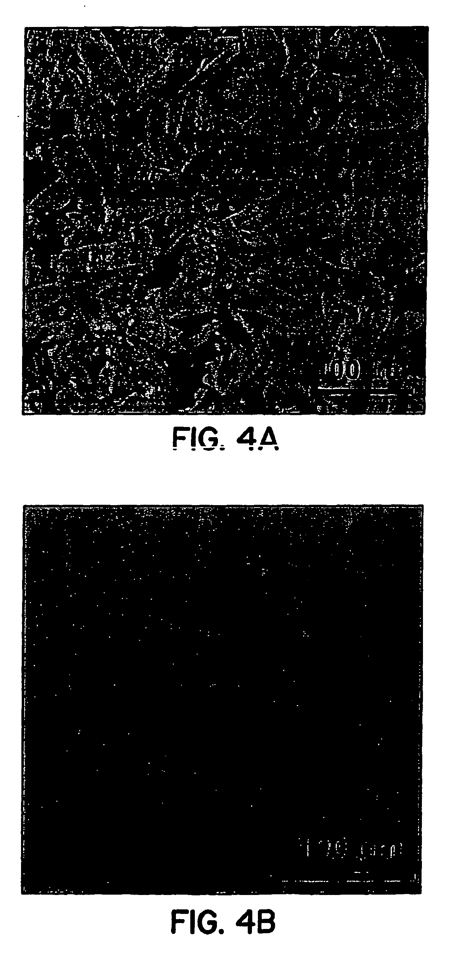

At room temperature, equal volumes of CaCl₂·2H₂O (1M) and Na₂HPO₄ (0.67M) were reacted. A calcium phosphate powder was obtained by filtering and drying the precipitate. The calcium phosphate powder was then analyzed under FTIR and SEM, which indicated that dicalcium phosphate (DCPD) formed. FTIR spectra were acquired using a Bruker Vertex 70 spectrometer (Bruker Optics Inc., Billerica, MA, USA), and SEM images were acquired using a LEO 1550 Variable Pressure field emission SEM (Carl Zeiss SMT Inc., Thornwood, NY, USA). Fig. 3A is the FTIR spectra of the calcium phosphate material. Fig. 4A is the SEM image of the calcium phosphate material.

### EXAMPLE 1B

At room temperature, equal volumes of CₐCl₂·2H₂O (1M) and Na₂HPO₄ (0.67M) were reacted. The resulting calcium phosphate dispersion was adjusted to a pH between 10 and 12 using 1 M NaOH. A calcium phosphate powder was obtained by filtering and drying the precipitate. The calcium phosphate powder was analyzed under FTIR and SEM, which indicated that calcium-deficient apatite formed. Fig. 3B is the FTIR spectra of the calcium phosphate material. Fig. 4B is the SEM image of the calcium phosphate material. Thus, by controlling the pH of the calcium phosphate material, calcium-deficient apatite was formed.

### EXAMPLE 2

A first mixture containing 10 w/w% PEGDMA macromers (synthesized as reported in Lin-Gibson et al. Biomacromolecules, 2004, 5, 1280-1287) having a molecular weight between about 2000 and 5000 g/mol, a 1 M solution of CaCl₂•2H₂O, and 0.05-1 w/w% (relative to concentration of PEGDMA) IRGACURE 2959 brand photoinitiator (Ciba Specialty Chemicals) was prepared. A second mixture containing 10 w/w% PEGDMA macromers, a 0.67M solution of Na₂HPO₄, and 0.05-1 w/w% (relative to concentration of PEGDMA) IRGACURE 2959 brand photoinitiator was also prepared.

40 µL of the first mixture was injected into a cylindrical mold having a height of 3 mm and a thickness of 6 mm. 40 µL of the second mixture was then injected into the mold. A calcium phosphate dispersion formed rapidly. The calcium phosphate dispersion was then exposed to a long wavelength UV source (365 nm, 300 µW/cm2) tor 10-30 minutes to crosslink the hydrogel to form a mineralized hydrogel.

### EXAMPLE 3

A first mixture was prepared by adding 10 w/w% poly(vinyl alcohol) ("PVA") polymer having a molecular weight of about 140,000 g/mol and a 1 M dispersion of CaCl₂•2H₂O to an aqueous dispersion of 25 w/w% dimethyl sulfoxide ("DMSO"). A second mixture was prepared by adding 10 w/w% of the same PVA polymer and a 0.67 of dispersion of Na₂HPO₄ to an aqueous dispersion of 25 w/w% DMSO.

The first and second mixtures were separately loaded into a DUO-PAK brand twist-lock cartridge having a 3/16 in tip (available from McMaster-Carr). The cartridge was connected to a dispensing gun, and the first and second mixtures were injected from the dispensing gun, through a static mixer (available from McMaster-Carr), and into a mold. The mold can be subjected to a conventional freeze-thaw cycle or other conventional crosslinking technique to crosslink the hydrogel to form the mineralized hydrogel.

### EXAMPLE 4

0.05 g IRGACURE^{R} 2959 brand photoinitiator (2-Hydroxy-1-[4-(2-hydroxyethoxy) phenyl]-2-methyl-1-propanone, Ciba Specialty Chemicals) was dissolved in 5 mL poly(ethylene glycol) diacrylate (PEGDA, Aldrich Inc., Mn -575, water soluble). The resulting PEGDA solution was mixed with 5mL 1M CaCl₂ to form a a first mixture. A 0.67M solution of Na .HPO was also prepared to form a second mixture. The second mixture was added dropwise to the first mixture under moderate stirring on a magnetic stirring plate (speed 3-4 in a 0-10 scale) to form a dispersion. Stirring of the dispersion was continued for another 15-30 min. The dispersion was then transferred to several UHMWPE cylinder molds having a diameter of 0.25 inch and a thickness of 0.25 inch. The molds were then positioned on a piece of glass and covered with a Mylar film. Photopolymerization of the dispersion was carried out for 3 min in a UV box (LOCTITE^{R} ZETA^{R} 7411-S UV Flood System, with a lamp power of 400 W and optimized wavelength between 315 and 400 nm) to form a mineralized hydrogel. After being removed from the molds, several gel specimens were soaked in a 1M NaOH solution. Control gel samples were prepared in the same matter except that no Ca and phosphate derivatives were included.

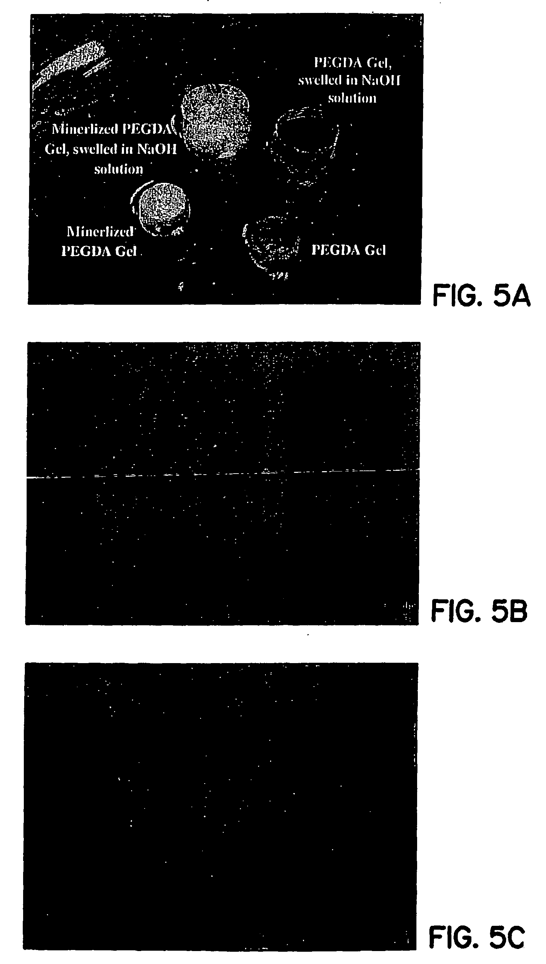

FIGS. 5A-5C show images of several samples formed as reported above. Fig. 5A shows two mineralized samples and two control samples. One mineralized sample and one control sample were soaked in NaOH. All four samples swelled when contacted with water, with the NaOH-soaked samples swelling to a greater degree.

FIGS. 5B-5C show microscopic views of a control sample (5A) and a mineralized sample (5B) obtained by light microscopy using a Zeiss Stemi 2000-C microscope (Carl Zeiss SMT Inc., Thornwood, NY, USA ). Notably, a substantially uniform dispersion of a calcium phosphate mineral is visible in the mineralized sample.

Alternatively, a mineralized hydrogel is prepared according to Example 4 except that the calcium phosphate dispersion is centrifuged prior to being added to the mold. The resulting mineralized hydrogel contains a gradient of calcium phosphate minerals.

### EXAMPLE 5

Poly(ethylene glycol) dimethacrylate (PEGDMA, Mn ~4000, water soluble) macromers were synthesized as reported in the literature (Lin-Gibson et al. Biomacromolecules, 2004, 5, 1280-1287). A first mixture was prepared by dissolving 0.28 g PEGDMA and 0.0003 g (~0.1 wt% relative to PEGDMA macromers) IRGACURE^{R} 2959 brand photoinitiator in 1.0 mL 1 M CaCl2 solution. A second mixture was provided by preparing a 1.0 mL 0.67M solution of Na₂HPO₄. The second mixture was added dropwise to the first mixture under moderate stirring on a magnetic stirring plate (speed 3-4 in a 0-10 scale) to form a calcium phosphate dispersion. The calcium phosphate dispersion was then stirred for another 15-30 min. A 10 wt% photoinitiated PEGDMA solution was prepared by dissolving 0.2 g PEGDMA in 1.8 g deionized water, along with 0.0002 g (0.1 wt% relative to PEGDMA macromers) Irgacure 2959 brand photoinitiator (2-Hydroxy-1-[4-(2-hydroxyethoxy) phenyl]-2-methyl-1-propanone, Ciba Specialty Chemicals). No calcium or phosphate materials were added to the 10 wt% photoinitiated PEGDMA solution. Several UHMWPE cylinder molds having a diameter of 0.25 inch and a thickness of 0.25 inch were positioned on a piece of glass and partially filled with the 10 wt% photoinitiated PEGDMA solution. The molds were then covered with a Mylar film. Photopolymerization of the partially filled molds was carried out for 4 min in a UV box (LOCTITE ZETA 7411-S UV Flood System, with a lamp power of 400 W and optimized wavelength between 315 and 400 nm) to form a PEGDMA hydrogel. The calcium phosphate dispersion was then added over the crosslinked PEGDMA hydrogel to fill up the molds. A Mylar film was again applied to cover the filled molds. Photopolymerization of the calcium phosphate dispersion was again carried out under the same conditions to form a composite hydrogel having bottom layer of PEGDMA hydrogel and the top layer of mineralized PEGDMA hydrogel.

A first control sample was prepared in the manner described above except that the molds were filled completely with the calcium phosphate dispersion and crosslinked to form a mineralized hydrogel. A second control sample was formed in the same manner except that the molds were filled completely with the 10 wt% photoinitiated PEGDMA solution and crosslinked to form a PEGDMA hydrogel.

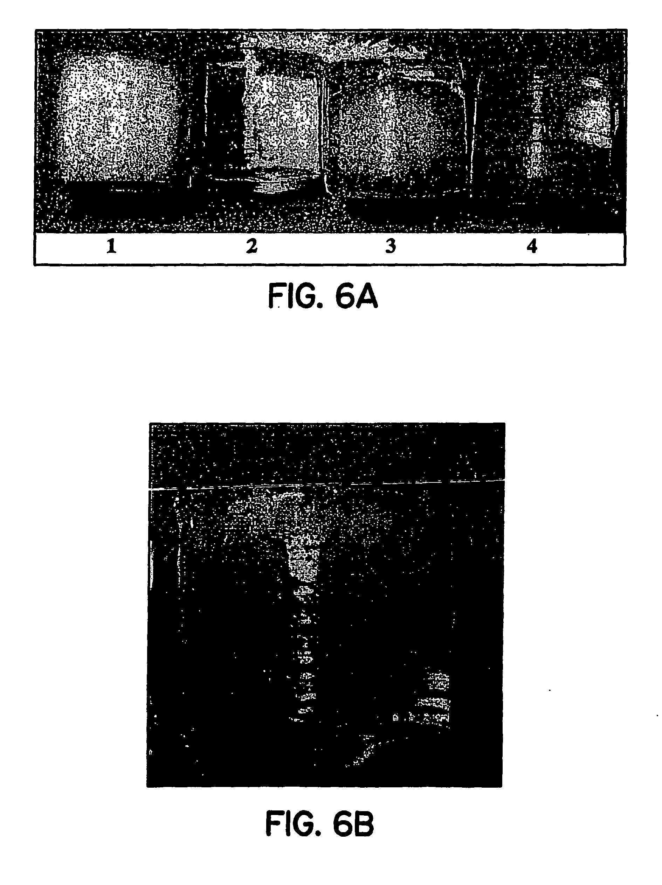

FIGS. 6A-B shows images of several samples formed as reported above. Samples 1 and 2 are images of the first and second control samples, respectively. Specimens 3 and 4 are two-layer composite hydrogels with varying thicknesses of mineralized PEGDMA hydrogel layers and PEGDMA hydrogel layers.

FIG. 6B show a close-up image of a two-layer sample with a top layer of mineralized PEGDMA hydrogel and a bottom layer of PEGDMA hydrogel.

Alternately, a layered hydrogel can be prepared as described above in Example 5, except that the calcium phosphate dispersion is added to the mold and photopolymerized first, followed by the addition and photopolymerization of the PEGDMA solution such that the bottom layer is mineralized PEGDMA hydrogel and the top layer is PEGDMA hydrogel.

### EXAMPLE 6

Calcium phosphate minerals in the absence of a hydrogel are prepared as in Example 1, using the concentrations of calcium and phosphate and pH control to yield amorphous calcium phosphate as demonstrated by FTIR, XRD and SEM. The amorphous calcium phosphate can then be converted to calcium-deficient apatite using appropriate pH adjustments, as described above. Alternately, calcium phosphate minerals in the absence of a hydrogel can be prepared as in Example 1, but using the concentrations of Ca and phosphate and pH control to yield calcium-deficient apatite as demonstrated by FTIR, XRD and SEM, as described above.

### EXAMPLE 7

Prepare and combine first and second mixtures according to Example 2. Prior to crosslinking, combine the calcium phosphate dispersion with a phosphate buffered saline solution (pH about 7.4) for up to about 24 hours and periodically test dried samples under FTIR, XRD and SEM until the desired conversion of amorphous calcium phosphate to calcium-deficient apatite and/or other crystalline calcium phosphates is achieved. Also, identify the microstructure of the inside of a sample using light microscopy or similar methods. Further, test the mechanical properties of samples using conventional mechanical tests procedures according to ASTM standards.

### EXAMPLE 8

Prepare the first and second mixtures according to Example 2. Separately load each mixture into a DUO-PAK¹ cartridge with a 3/16 in. tip, which is connected to a dispensing gun, as described in Example 3. Dispense the first and second mixture from the dispensing gun, through a static mixture and into a mold or Petri dish. Crosslink via physical or chemical crosslinking and characterize the hydrogel using light microscopy, XRD, FTIR and SEM.

### EXAMPLE 9

Prepare a layered hydrogel as described in Example 5, except that a third mixture is prepared by dissolving 0.28 g PEGDMA and 0.0003 g (-1 wt% relative to PEGDMA macromers) IRGACURE^{R} 2959 brand photoinitiator in 1.0 mL 0.1M CaCl₂ solution. A 1.0 mL 0.067M solution of Na₂HPO₄ is also prepared to form a fourth mixture. The fourth mixture is added dropwise to the third mixture under moderate stirring on a magnetic stirring plate (speed 3-4 in a 0-10 scale) to form an additional calcium phosphate dispersion. This additional calcium phosphate dispersion is filled into a mold over the previously-formed two-layer hydrogel described in Example 5. The mold is then covered with a Mylar film and photopolymerized under the conditions described in Example 5 to form a three-layer gel with the top two layers providing a calcium phosphate concentration gradient. Alternatively the ordering of the layers can be modified such that the bottom two layers form a calcium phosphate gradient and the top layer is a PEGDMA gel.

While the present invention has been illustrated by the description of one or more embodiments thereof, and while the embodiments have been described in considerable detail, additional advantages and modifications will readily appear to those skilled in the art.

## Claims

1. A method for preparing a mineralized hydrogel including preparing a first mixture including a calcium derivative and a second mixture including a phosphate derivative, wherein at least one of the first and second mixtures further includes at least one hydrogel precursor, and combining the first mixture and the second mixture under conditions which form a mineralized hydrogel.

2. The method of claim 1, wherein the combining further includes crosslinking the hydrogel precursor.

3. A method for preparing a mineralized hydrogel comprising preparing a first mixture including a calcium derivative and a second mixture including a phosphate derivative, wherein at least one of the first and second mixtures further includes at least one hydrogel precursor, combining the first mixture and the second mixture to form a calcium phosphate dispersion containing the at least one hydrogel precursor, and crosslinking the hydrogel precursor in the calcium phosphate dispersion to form the mineralized hydrogel.

4. The method of any preceding claim wherein a photoinitiator is added to the first mixture, the second mixture or the calcium phosphate dispersion, and the crosslinking includes a photoinitiation step.

5. The method of any one of claims 1 to 3 wherein the crosslinking includes a photoinitiation step, a chemical crosslinking step, a physical crosslinking step, an irradiation step or combinations thereof.

6. A method of forming a mineralized hydrogel at a site including preparing a calcium phosphate dispersion by combining a first mixture including a calcium derivative, a second mixture including a phosphate derivative, and wherein at least one of the first and second mixtures further includes at least one hydrogel precursor, delivering either the calcium phosphate dispersion or the first and second mixtures to a site, and crosslinking the calcium phosphate dispersion at the site to form a mineralized hydrogel.

7. The method of claim 6 wherein the delivering includes delivering the first and second mixtures to the site, and the preparing step occurs at the site.

8. The method of either claim 6 or claim 7 wherein the crosslinking includes a chemical crosslinking or irradiation crosslinking step.

9. The method of any preceding claim wherein the calcium derivative is an anhydrous or hydrated calcium material and the phosphate derivative is an anhydrous or hydrated phosphate material.

10. The method of claim 9 wherein the calcium derivative is selected from CaCl₂ or Ca(NO₃)₂.

11. The method of either claim 9 or claim 10 wherein the phosphate derivative is selected from K₂HPO₄, Na₂HPO₄ or (NH₄)₂HPO₄.

12. The method of any preceding claim wherein the hydrogel precursor is selected from one or more of the following:
a precursor derived from a hydrogel;
a monomer, macromer, oligomers, homopolymer or copolymer of poly(vinyl alcohol), a poly(glycol), poly(ethylene glycol) dimethacrylate, poly(ethylene glycol) diacrylate, poly(hydroxyethyl methacrylate), poly(vinyl pyrrolidone), poly(acrylamide), poly(acrylic acid), hydrolyzed poly(acrylonitrile), poly(ethyleneimine), ethoxylated poly(ethyleneimine) or poly(allylamine);
a biopolymer selected from chitosan, agarose, hyaluronic acid, collagen or gelatine, a (semi) interpenetrating network hydrogel;
a peptide-, protein-, calcium-, or phosphate-modified monomers, oligomers, macromers, or polymers;
synthetic polypeptide hydrogels;
or derivatives, combinations or blends thereof.

13. The method of any preceding claim wherein the first mixture includes between 0.1 mM and 5M of the calcium derivative and the second mixture includes between 0.1mM and 5M of the phosphate derivative.

14. The method of any preceding claim wherein the ratio of calcium ions to phosphate ions in the calcium phosphate dispersion is between about 1:1 and about 2:1.

15. The method of any preceding claim wherein the hydrogel precursor is added to each of the first and second mixtures prior to forming the calcium phosphate dispersion.

16. The method of any preceding wherein the first and second mixtures include the same or different hydrogel precursor.

17. The method of any preceding claim further comprising the step of adjusting the pH or temperature of the calcium phosphate dispersion or the mineralized hydrogel.

## Patentansprüche

1. Verfahren zur Herstellung eines mineralisierten Hydrogels, das das Herstellen einer ersten Mischung, die ein Calciumderivat umfasst, und einer zweiten Mischung, die ein Phosphatderivat umfasst, wobei mindestens eine der ersten und zweiten Mischungen des Weiteren mindestens einen Hydrogelvorläufer umfasst, und das Kombinieren der ersten Mischung und der zweiten Mischung unter Bedingungen, unter denen ein mineralisiertes Hydrogel gebildet wird, umfasst.

2. Verfahren nach Anspruch 1, wobei das Kombinieren des Weiteren das Vernetzen des Hydrogelvorläufers umfasst.

3. Verfahren zur Herstellung eines mineralisierten Hydrogels, das das Herstellen einer ersten Mischung, die ein Calciumderivat umfasst, und einer zweiten Mischung, die ein Phosphatderivat umfasst, wobei mindestens eine der ersten und zweiten Mischungen des Weiteren mindestens einen Hydrogelvorläufer umfasst, das Kombinieren der ersten Mischung und der zweiten Mischung unter Bildung einer Calciumphosphatdispersion, die den mindestens einen Hydrogelvorläufer enthält, und das Vernetzen des Hydrogelvorläufers in der Calciumphosphatdispersion unter Bildung eines mineralisiertes Hydrogels umfasst.

4. Verfahren nach einem der vorhergehenden Ansprüche, wobei ein Fotoinitiator der ersten Mischung, der zweiten Mischung oder der Calciumphosphatdispersion hinzugegeben wird und das Vernetzen einen Fotoinitiierungsschritt umfasst.

5. Verfahren nach einem der Ansprüche 1 bis 3, wobei das Vernetzen einen Fotoinitiierungsschritt, einen chemischen Vernetzungsschritt, einen physikalischen Vernetzungsschritt, einen Bestrahlungsschritt oder Kombinationen derselben umfasst.

6. Verfahren zum Bilden eines mineralisierten Hydrogels an einer Stelle, das das Herstellen einer Calciumphosphatdispersion durch Kombinieren einer ersten Mischung, die ein Calciumderivat umfasst, einer zweiten Mischung, die ein Phosphatderivat umfasst, und wobei mindestens eine der ersten und zweiten Mischungen des Weiteren mindestens einen Hydrogelvorläufer umfasst, das Zuführen entweder der Calciumphosphatdispersion oder der ersten und zweiten Mischungen zu einer Stelle und das Vernetzen der Calciumphosphatdispersion an der Stelle unter Bildung eines mineralisierten Hydrogels umfasst.

7. Verfahren nach Anspruch 6, wobei das Zuführen das Zuführen der ersten und zweiten Mischungen zu der Stelle umfasst und der Herstellungsschritt an der Stelle erfolgt.

8. Verfahren nach entweder Anspruch 6 oder Anspruch 7, wobei das Vernetzen einen chemischen Vernetzungs- oder Bestrahlungsvernetzungsschritt umfasst.

9. Verfahren nach einem der vorhergehenden Ansprüche, wobei das Calciumderivat ein wasserfreies oder hydratisiertes Calciummaterial und das Phosphatderivat ein wasserfreies oder hydratisiertes Phosphatmaterial ist.

10. Verfahren nach Anspruch 9, wobei das Calciumderivat unter CaCl₂ oder Ca(NO₃)₂ ausgewählt wird.

11. Verfahren nach entweder Anspruch 9 oder Anspruch 10, wobei das Phosphatderivat unter K₂HPO₄, Na₂HPO₄ oder (NH₄)₂HPO₄ ausgewählt wird.

12. Verfahren nach einem der vorhergehenden Ansprüche, wobei der Hydrogelvorläufer aus einem oder unter mehreren der Folgenden ausgewählt wird:
einem von einem Hydrogel derivatisierten Vorläufer;
einem Monomer, Makromer, Oligomeren, Homopolymer oder Copolymer von Poly(vinylalkohol), einem Poly(glykol), Poly(ethylenglykol)dimethacrylat, Poly(ethylenglykol)diacrylat, Poly(hydroxyethylmethacrylat), Poly(vinylpyyrolidon), Poly(acrylamid), Poly(acrylsäure), hydrolysiertem Poly(acrylnitril), Poly(ethylenimin), ethoxyliertem Poly(ethylenimin) oder Poly(allylamin);
einem Biopolymer ausgewählt unter Chitosan, Agarose, Hyaluronsäure, Collagen oder Gelatine, einem (semi-)interpenetrierenden Netzwerk-Hydrogel;
peptid-, protein-, calcium- oder phosphatmodifizierten Monomeren, Oligomeren, Makromeren oder Polymeren;
synthetischen Polypeptid-Hydrogelen;
oder Derivaten, Kombinationen oder Mischungen derselben.

13. Verfahren nach einem der vorhergehenden Ansprüche, wobei die erste Mischung 0,1 mM bis 5 M des Calciumderivates und die zweite Mischung 0,1 mM bis 5 M des Phosphatderivats umfasst.

14. Verfahren nach einem der vorhergehenden Ansprüche , wobei das Verhältnis von Calciumionen zu Phosphationen in der Calciumphosphatdispersion zwischen etwa 1 : 1 und etwa 2 : 1 liegt.

15. Verfahren nach einem der vorhergehenden Ansprüche, wobei der Hydrogelvorläufer jeder der ersten und zweiten Mischungen vor dem Bilden der Calciumphosphatdispersion hinzugesetzt wird.

16. Verfahren nach einem der vorhergehenden Ansprüche, wobei die ersten und zweiten Mischungen denselben oder einen anderen Hydrogelvorläufer umfassen.

17. Verfahren nach einem der vorhergehenden Ansprüche, des Weiteren umfassend den Schritt des Einstellens des pH-Werts oder der Temperatur der Calciumphosphatdispersion oder des mineralisierten Hydrogels.

## Revendications

1. Procédé pour préparer un hydrogel minéralisé comprenant la préparation d'un premier mélange incluant un dérivé de calcium et un deuxième mélange incluant un dérivé de phosphate, dans lequel au moins un du premier mélange et du deuxième mélange comprend en outre au moins un précurseur d'hydrogel, et la combinaison des premier et deuxième mélanges dans des conditions qui forment un hydrogel minéralisé.

2. Procédé selon la revendication 1, dans lequel la combinaison comprend en outre une réticulation du précurseur d'hydrogel.

3. Procédé pour préparer un hydrogel minéralisé comprenant la préparation d'un premier mélange incluant un dérivé de calcium, et d'un deuxième mélange incluant un dérivé de phosphate, dans lequel au moins un du premier mélange et du deuxième mélange comprend en outre au moins un précurseur d'hydrogel, la combinaison des premier et deuxième mélanges pour former une dispersion de phosphate de calcium contenant l'au moins un précurseur d'hydrogel, et la réticulation du précurseur d'hydrogel dans la dispersion de phosphate de calcium pour former l'hydrogel minéralisé.

4. Procédé selon l'une quelconque des revendications précédentes, dans lequel un photoinitiateur est ajouté au premier mélange, au deuxième mélange ou à la dispersion de phosphate de calcium, et la réticulation comprend une étape de photoinitiation.

5. Procédé selon l'une quelconque des revendications 1 à 3, dans lequel la réticulation comprend une étape de photoinitiation, une étape de réticulation chimique, une étape de réticulation physique, une étape d'irradiation ou des combinaisons des précédentes.

6. Procédé pour former un hydrogel minéralisé à un site comprenant la préparation d'une dispersion de phosphate de calcium en combinant un premier mélange incluant un dérivé de calcium et un deuxième mélange incluant un dérivé de phosphate, et dans lequel au moins un du premier mélange et du deuxième mélange comprend en outre au moins un précurseur d'hydrogel, fournissant soit la dispersion de phosphate de calcium ou les premier et deuxième mélanges à un site, et la réticulation de la dispersion de phosphate de calcium au site pour former un hydrogel minéralisé.

7. Procédé selon la revendication 6, dans lequel la fourniture comprend la fourniture des premier et deuxième mélanges au site, et l'étape de préparation a lieu au site.

8. Procédé selon la revendication 6 ou 7, dans lequel la réticulation comprend une étape de réticulation chimique ou de réticulation par irradiation.

9. Procédé selon l'une quelconque des revendications précédentes, dans lequel le dérivé de calcium est un matériau de calcium anhydre ou hydraté et le dérivé de phosphate est un matériau de phosphate anhydre ou hydraté.

10. Procédé selon la revendication 9, dans lequel le dérivé de calcium est sélectionné parmi CaCl₂ ou Ca(NO₃)₂.

11. Procédé selon la revendication 9 ou 10, dans lequel le dérivé de phosphate est sélectionné parmi K₂HPO₄, Na₂HPO₄ ou (NH₄)₂HPO₄.

12. Procédé selon l'une quelconque des revendications précédentes, dans lequel le précurseur d'hydrogel est sélectionné parmi :
Un précurseur dérivé d'un hydrogel ;
un monomère, un macromère, des oligomères, un homopolymère ou un copolymère de poly(vinyl alcohol), poly(glycol), poly(éthylène glycol) diméthacrylate, poly(éthylène glycol) diacrylate, poly(hydroxyéthyl méthacrylate), poly(vinyl pyrrolidone), poly(acrylamide), poly(acide acrylique), poly(acrylonitrile) hydrolysé, poly(éthylèneimine), poly(éthylèneimine) éthoxylée ou poly(allylamine) ;
un biopolymère sélectionné parmi le chitosane, l'agarose, l'acide hyaluronique, le collagène ou la gélatine, un hydrogel à réseaux (semi)interpénétrants ;
des monomères, oligomères, macromères ou polymères modifiés par des peptides, protéines, du calcium ou du phosphate ;
des hydrogels polypeptides synthétiques ;
ou bien des dérivés, combinaisons ou mélanges des précédents.

13. Procédé selon l'une quelconque des revendications précédentes, dans lequel le premier mélange contient entre 0,1 mM et 5 M du dérivé de calcium et le deuxième mélange contient entre 0,1 mM et 5 M du dérivé de phosphate.

14. Procédé selon l'une quelconque des revendications précédentes, dans lequel le ratio des ions calcium/ions phosphate dans la dispersion de phosphate de calcium se situe entre 1:1 environ et 2:1 environ.

15. Procédé selon l'une quelconque des revendications précédentes, dans lequel le précurseur d'hydrogel est ajouté à chacun des premier et deuxième mélanges avant la formation de la dispersion de phosphate de calcium.

16. Procédé selon l'une quelconque des revendications précédentes, dans lequel les premier et deuxième mélanges incluent un précurseur d'hydrogel identique ou différent.

17. Procédé selon l'une quelconque des revendications précédentes, comprenant en outre l'étape d'ajustement du pH ou de la température de la dispersion de phosphate de calcium ou de l'hydrogel minéralisé.
